# EUROPEAN PATENT APPLICATION

(11) **EP 3 165 218 A1**
(43) Date of publication of application: **10.05.2017**
(21) Application number: 15193413.0
(22) Date of filing: 06.11.2015
(51) Int. Cl.: A61K 9/00, A61K 8/35, A61K 9/16, A61K 9/20, A61K 31/122

(54) **WATER DISPERSIBLE GRANULATES CONTAINING OXIDIZED OR REDUCED FORMS OF COENZYME Q10**

(71) Applicant: INDENA S.p.A., 20139 Milano (IT)
(72) Inventor: RONCHI, Massimo, 20139 MILANO (IT); DALLERA, Enrico, 20139 MILANO (IT); FRATTINI, Elisabetta, 20139 MILANO (IT)
(74) Representative: Bianchetti Bracco Minoja S.r.l.

(57) **Abstract**

The invention discloses water dispersible powder granulates comprising Coenzyme Q₁₀ in its oxidized (Ubiquinone) and/or reduced form (Ubiquinol), at least one surfactant other than sucrose esters and at least one freely soluble carrier, characterised by fast water dispersion, increased solubility, faster dissolution rate and hence better oral bioavailability.

## Description

The present invention concerns water dispersible powder granulates comprising Coenzyme Q₁₀ in its oxidized (Ubiquinone) and/or reduced form (Ubiquinol), at least one surfactant and at least one freely soluble carrier, a process for their preparation and pharmaceutical, nutritional, dietary, veterinary or cosmetic compositions containing them.

### Background of the invention

Coenzyme Q₁₀, also known as ubidecarenone or ubiquinone, is a lipophilic endogenous substance present in most eukaryotic cells, primarily concentrated in mitochondria. Coenzyme Q₁₀ participates to the mitochondrial oxido-reductive reactions of electron transport chain for the generation of energy, in the form of Adenosine Triphosphate (ATP). Coenzyme Q₁₀ can exist in three oxidation states: (1) the fully reduced form (ubiquinol), (2) the radical intermediate form (semiquinone), and (3) the fully oxidized form (ubiquinone). Coenzyme Q₁₀ exists in human body in all these forms and a physiological equilibrium between the oxidized and the reduced form is maintained.

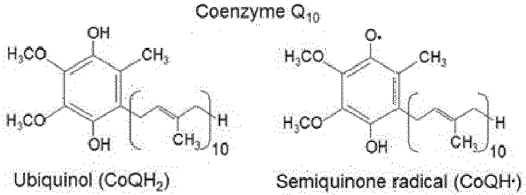

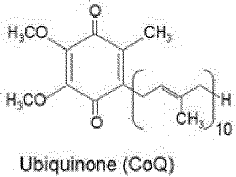

The name ubidecarenone or ubiquinone (or ubiquinol for the reduced form) is related to the fact that Coenzyme Q₁₀ is ubiquitously distributed in the organs of the human body, but it is primarily concentrated in organs with a higher energy requirement, like heart, liver and kidney.

The name ubiquinone (or ubiquinol for the reduced form) also refers to its quinone (or hydroquinone) structure, while the number 10 is the number of isoprenyl units in its tail.

Coenzyme Q₁₀, in both its oxidized and reduced form, can also be introduced in our body with the diet, even if the contribution of endogenous Coenzyme Q₁₀ to its physiological plasma levels has not been clarified. The richer sources of dietary Coenzyme Q₁₀ include meat, poultry and fish, while fruits, vegetables and eggs are limited sources of Coenzyme Q₁₀.

As Coenzyme Q₁₀ deficiency is rare, oral coenzyme Q₁₀ supplementation is mainly used to maintain homeostasis of the body, to promote heart health, as energy booster and also to treat different diseases like ageing, periodontal disease, impaired memory, fatigue, coronary disease, high blood pressure, immune system impairment.

Coenzyme Q₁₀ supplementation can be particularly useful for elderly people, as the tissue and plasma physiological levels of Coenzyme Q₁₀ are reported to decline with age.

Ubiquinone is a yellow-orange crystalline powder, with a low melting point (about 50°C), while Ubiquinol is an off-white powder with an even lower melting point (about 47-48°C).

The activity of Coenzyme Q₁₀ supplementation can be strongly limited by its poor pharmacokinetic properties and, in particular, by its very low oral bioavailability, due to the fact that Coenzyme Q₁₀ is often commercialized in a totally crystalline form and it is characterized by high lipophilicity and relatively high molecular weight.

Several formulations approaches have been applied to promote CoQ₁₀ bioavailability, including self-emulsifying delivery systems, inclusion in cyclodextrins, solid dispersions, lipophilic formulations, microspheres, nanoparticles, etc., but absorption of Coenzyme Q₁₀ into the systemic circulation still remains a challenge.

Moreover the hydrophobic nature of Ubiquinone and Ubiquinol strongly limits the applicable formulation approaches and, in particular, the possibility to incorporate Ubiquinone and/or Ubiquinol in water based formulations like beverages, ready-to- use or extemporary liquid dosage forms. These formulation approaches can be particularly suitable for elderly people due to their reduced swallowing capability.

### Description of the invention

It has now been found that water dispersible powder granulates of Coenzyme Q₁₀, characterised by fast water dispersion, increased solubility, faster dissolution rate and hence better oral bioavailability, may be obtained in the presence of suitable auxiliary ingredients comprising at least one surfactant and at least one freely soluble carrier.

In particular, according to the invention, Coenzyme Q₁₀, in its reduced and/or oxidized form, is homogeneously and finely layered on at least one freely soluble carrier in intimate contact with at least one selected surfactant.

The invention accordingly provides water dispersible powder granulates comprising Coenzyme Q₁₀ in its oxidized (Ubiquinone) and/or reduced form (Ubiquinol), at least one surfactant and at least one freely soluble carrier, with the proviso that said surfactant is not a sucrose ester. The invention also concerns a process for the preparation of said granulates and pharmaceutical, nutritional, dietary, veterinary or cosmetic compositions containing them.

Examples of suitable surfactants include lecithins, polyoxyethylene alkyl ethers, polyoxyethylene castor oil derivatives, polyoxyethylene sorbitan fatty acid esters, polyoxyethylene stearates, polyglycerides, propylene glycol mono- and di-laurate, sorbitan fatty acid esters, , polyoxyl 15 hydroxystearate, D-α-tocopheryl polyethylene glycol succinate.

Lecithins, polysorbates and polyoxyethylene castor oil derivatives are preferred. More preferably, the surfactant is lecithin, either alone or in combination with at least another surfactant as defined above, always excluding sucrose esters.

Examples of freely soluble carriers include glucose, sucrose, fructose, mannitol, sorbitol, isomalt, maltitol, maltodextrins, cyclodextrins, maltose, lactose, polydextrose, dextrin, dextranes, dextrose, inulin. Maltodextrin, mannitol and isomalt are preferred.

The fine dimension of the dispersed Coenzyme Q₁₀ particles, the local high surfactant concentration, which locally increases the wettability and the solubility of Coenzyme Q₁₀ and the fast dissolution rate of the freely soluble carrier, determine the effective and homogeneous water dispersion, the increased solubility and the faster dissolution rate of Coenzyme Q₁₀.

Other ingredients can be added to the granulates of the invention with the purpose to further improve the performance and/or to stabilize the water dispersion of Coenzyme Q₁₀. These auxiliary ingredients include cellulosic derivatives (carboxymethyl-cellulose, hydroxymethyl cellulose, hydroxypropyl cellulose, hydroxypropylmethyl cellulose), natural gums (acacia gum, arabic gum carnauba gum, guar gum, xanthan gum), alginic acid, pectin, povidone, starch and starch derivatives.

Effective disintegrants can be also added to further promote a fast disintegration and dissolution upon contact with water. These auxiliary ingredients include polyvinylpolypirrolidone, sodium starch glycolate, sodium croscarmellose.

Antifoaming agents, such as Simethicone can also be added to avoid the formation of stable foams during water dispersion.

The water dispersible powder granulates of the invention are also characterized by suitable technological properties, including good flowability and sufficiently high apparent density to be easily incorporated either in different pharmaceutical, nutraceutical, veterinary and cosmetic formulations or in conventional foods.

The solid water dispersible granulates of the invention are prepared by dispersing and solubilizing Coenzyme Q₁₀ and surfactants in a suitable solvent, preferably selected from ethyl alcohol, isopropyl alcohol, n-propyl alcohol, n-butanol, ethyl acetate, acetone. The Coenzyme Q₁₀, surfactant solution/suspension is added or sprayed, preferably under mixing, to a freely soluble carrier. An intimate contact between the components is guaranteed by an efficient mixing of the mass. Other ingredients, having the purpose to improve the performances of the powdered formulations, can be added either to the Coenzyme Q₁₀ solution or to the soluble carrier.

After granulation, the powder formulation is isolated by removing the solvent under vacuum.

The obtained powder is then calibrated and eventually grinded to optimize its particle size distribution.

The powdered granulates of the invention can be prepared using well-known and easily accessible manufacturing procedures including, for instance low and high-shear wet granulation, fluid-bed granulation.

According to the invention, the water dispersible granulates contain a percentage by weight of Coenzyme Q10, in its oxidized and/or reduced form, ranging between 5 and 30% by weight, more preferably between 10 and 20% by weight.

The Coenzyme Q₁₀/surfactant(s) ratio is preferably from 0,2 to 10, more preferably from 0,5 to 5.

The Coenzyme Q₁₀/freely dissolving carrier(s) ratio is preferably from 0,05 to 2, more preferably from 0,1 to 0,5.

A calorimetric analysis by Differential Scanning Calorimetry (DSC) was also performed to calculate, on the basis of the reduction of the enthalpy of fusion (J/g), the degree of amorphization of Coenzyme Q₁₀ in the solid dispersion, in comparison with the totally crystalline Coenzyme Q₁₀.

Another object of the invention concerns formulations for oral administration containing the granulates of the invention. To this purpose, pharmaceutically and food acceptable ingredients, like disintegrants, lubricants, binders, coating agents, colorants, absorption enhancers, solubilizing agents, stabilizers, flavors, sweeteners, antiseptics, preservatives, antioxidants and the like can be used.

Examples of dosage forms of the formulations of the invention include chewable tablets, capsules, soft gelatin capsules, hard gelatin capsules, caplets, lozenges, chewable lozenges, powder for reconstitution, extemporary or ready-to-use suspensions, health bars, confections, animal feeds, cereals, cereal coatings, beverages and combinations thereof.

Another object of the invention concerns formulations for topical administration containing the granulate of the invention. To this purpose cosmetic acceptable ingredients can be used.

The preparation of the above dosage forms are well known.

The composition for both oral and topical administration of the invention can be used for a variety of purposes for improving the quality of life of humans, including the prevention and treatment for various diseases, reduction of side reactions, promotion of recovery from disease and the like, and can also be used for the purpose of maintaining and promoting daily health and the like.

The dosage of the composition for oral administration of the invention is preferably 1 to 1200 mg per day for a human, based on the amount of Coenzyme Q₁₀, more preferably 10 to 800 mg, and from the viewpoint of routine ingestion and onset of effects, it is particularly preferably 30 to 500 mg. The above-described daily amount can be taken at one time or in several divided portions.

The water dispersability of the granulate of the invention was evaluated visually in comparison with unformulated Coenzyme Q10 administered as crystal powder. A quantity of the granulate of the invention corresponding to 50 mg of Coenzyme Q₁₀ was dispersed in about 40 ml of tap water at room temperature. The product formed a very fine and homogeneous cloudy dispersion under mild agitation and no precipitation occurred. When the same evaluation was performed with 50 mg of crystalline Coenzyme Q10, the powder remained on the surface of water, due to its very poor wettability, even under strong and prolonged agitation.

The improved water solubility of Coenzyme Q₁₀ from the particulate composition of the invention was also evaluated confirmed by a solubility test performed in comparison with crystalline Coenzyme Q₁₀, as described in Examples 4 and 5.

### EXAMPLES

### Example 1 Preparation of the water dispersible Coenzyme Q₁₀ formulation

3,90 Kg of Isomalt was charged in a high shear mixer.

0,10 g of sunflower lecithin, 0,50 Kg of Polysorbate 80 and 0,50 Kg of Coenzyme Q₁₀ were solubilized in 1,3 Kg of ethyl alcohol under mixing at 45°C in the dark to prevent Coenzyme Q₁₀ degradation.

The solution was then cooled to room temperature and slowly added to isomalt in the high shear mixer. At the end of the wetting phase, the wet mass was kneaded for about 10 minutes under mixing (impeller: 400 rpm). At the end of the kneading step, the solvent was removed under vacuum for about 2,5 hours. At the end of the drying step the product temperature was about 45°C and the residual of ethyl alcohol was lower than 10000 ppm.

The resulting solid was calibrated through a 30 mesh screen to obtain a yellow-orange solid.

### Example 2 Preparation of the water dispersible Coenzyme Q10 formulation

800 g of Maltodextrins and 760 g of Mannitol were charged in a high shear mixer and blended for 5 minutes at 300 rpm.

120 g of fluid soy lecithin and 120 g of PEG-40 hydrogenated castor oil were solubilized in 600 g of ethyl acetate. 200 g of Ubiquinol was added to the obtained solution under stirring under nitrogen flux, until complete solubilization.

The obtain solution was slowly added to the Maltodextrins/mannitol blend in the high shear mixer under stirring. The wet mass was kneaded for 10 minutes to allow an intimate contact between the ingredients. All these operation were performed under nitrogen atmosphere. At the end of the kneading step the wet mass was dried under vacuum keeping the product temperature under 45°C.

The dried powder was calibrated through a 30 mesh screen to obtain a suitable granulometry.

### Example 3 Preparation of the water dispersible Coenzyme Q10 formulation

3,65 Kg of Isomalt was charged in a high shear mixer.

0,10 g of sunflower lecithin, 0,50 Kg of Polysorbate 80 and 0,75 Kg of Coenzyme Q₁₀ were solubilized in 1,3 Kg of ethyl alcohol under mixing at 45°C in the dark to prevent Coenzyme Q₁₀ degradation.

The solution was then cooled to room temperature and slowly added to isomalt in the high shear mixer. At the end of the wetting phase, the wet mass was kneaded for about 10 minutes under mixing (impeller: 400 rpm). At the end of the kneading step, the solvent was removed under vacuum for about 2,5 hours. At the end of the drying step the product temperature was about 45°C and the residual of ethyl alcohol was lower than 10000 ppm.

The resulting solid was calibrated through a 30 mesh screen to obtain a yellow-orange solid.

### Example 4 Solubility test

An amount of the formulation described in Example 1 corresponding to 40 mg of Coenzyme Q₁₀ was added to 20 ml of purified water in a glass container (corresponding to a final concentration of 2 mg/ml of Coenzyme Q₁₀). Even after vigorous and prolonged stirring, undissolved material was observed (saturated solution). The obtained suspension was kept under magnetic stirring during the experiment. Aliquots of the suspension were sampled at defined times. These aliquots of the suspension were filtered with a PTFE syringe filter (0,20 µm) to remove the undissolved material. The solutions were then analyzed by UPLC to determine Coenzyme Q₁₀ content. The solubility test was performed in parallel, in the same experimental conditions, on pure crystalline Coenzyme Q₁₀ powder.

The solubility curves, reported in the following figure, clearly shows the capacity of the formulation described in example 1 to promote Coenzyme Q₁₀ solubility, if compared with pure crystalline coenzyme Q₁₀.

### Example 5 Solubility test

An amount of the formulation described in Example 3 corresponding to 40 mg of Coenzyme Q₁₀ was added to 20 ml of purified water in a glass container (corresponding to a final concentration of 2 mg/ml of Coenzyme Q₁₀). Even after vigorous and prolonged stirring, undissolved material was observed (saturated solution). The obtained suspension was kept under magnetic stirring during the experiment. Aliquots of the suspension were sampled at defined times. These aliquots of the suspension were filtered with a PTFE syringe filter (0,20 µm) to remove the undissolved material. The solutions were then analyzed by UPLC to determine Coenzyme Q₁₀ content. The solubility test was performed in parallel, in the same experimental conditions, on pure crystalline Coenzyme Q₁₀ powder.

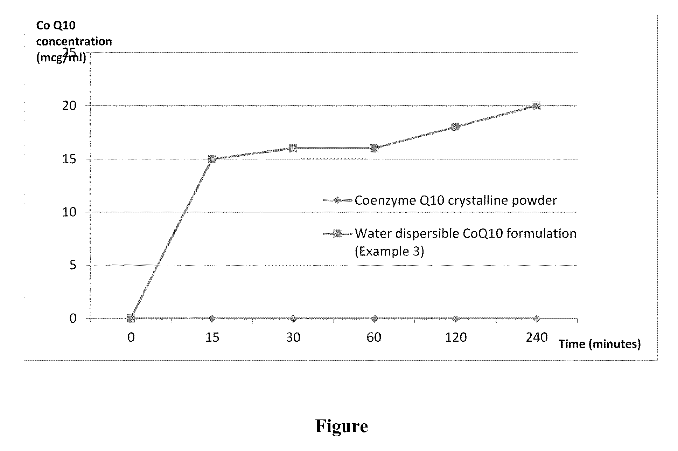

The solubility curves, reported in figure 1, clearly shows the capacity of the formulation described in example 1 to promote Coenzyme Q₁₀ solubility, if compared with pure crystalline coenzyme Q₁₀.

### Example 6 Formulations containing the water dispersible Coenzyme Q₁₀ 10% powder (tablets)

| | |
|---|---|
| Water dispersible Coenzyme Q₁₀ powder (Example 1) | 500,0 mg |
| Microcrystalline cellulose | 200,0 mg |
| Dicalcium phosphate anhydrous | 154,0 mg |
| Sodium croscarmellose | 30,0 mg |
| Silicon dioxide | 8,0 mg |
| Magnesium behenate | 8,0 mg |

1,0 Kg of water dispersible Coenzyme Q₁₀ powder, 0,4 Kg of Microcrystalline cellulose (for direct compression), 0,308 Kg of Dicalcium phosphate anhydrous (for direct compression) and 0,06 Kg of Sodium croscarmellose were blended in a V-mixer for 10 minutes. 16 g of Silicon dioxide and 16 g of Magnesium stearate were added to the powder mixture and blended for addition 2 minutes. The obtain mixture was compressed in a single punch tabletting machine, equipped with a round concave punch with a diameter of 12 mm.

### Example 7 Formulations containing the water dispersible Coenzyme Q₁₀ 10% powder (size 0 hard gelatin capsules)

| | |
|---|---|
| Water dispersible Coenzyme Q₁₀ powder (Example 3) | 333,0 mg |
| Silicon dioxide | 5,0 mg |
| Magnesium stearate | 5,0 mg |

1,665 Kg of water dispersible Coenzyme Q₁₀ powder was blended with 25 g of Silicon dioxide and 25 g of Magnesium stearate in a V-mixer for 2 minutes. The mixture was filled in size 1 hard gelatin capsules.

## Claims

1. Water dispersible powder granulates comprising Coenzyme Q₁₀ in its oxidized (Ubiquinone) and/or reduced form (Ubiquinol), at least one surfactant and at least one freely soluble carrier, with the proviso that said surfactant is not a sucrose ester.

2. The water dispersible granulates according to claim 1 wherein the surfactant is selected from the group consisting of lecithin, polyoxyethylene alkyl ethers, polyoxyethylene castor oil derivatives, polyoxyethylene sorbitan fatty acid esters, polyoxyethylene stearates, polyglycerides, propylene glycol mono- and di-laurate, sorbitan fatty acid esters, polyoxyl 15 hydroxystearate, D-α-tocopheryl polyethylene glycol succinate.

3. The water dispersible granulates according to claim 2 wherein said surfactant is lecithin.

4. The water dispersible granulates according to claim 1 wherein the surfactants include lecithin and another surfactant, sucrose ester excluded.

5. The water dispersible granulates according to any one of claims from 1 to 4 wherein the freely soluble carrier is selected from the group consisting of glucose, sucrose, fructose, mannitol, sorbitol, isomalt, maltitol, maltodextrins, cyclodextrins, maltose, lactose, polydextrose, dextrin, dextrans, dextrose, inulin.

6. The water dispersible granulates according to any one of claims from 1 to 5 further comprising auxiliary ingredients selected from cellulosic derivatives, alginic acid, povidone, starch and starch derivatives, polyvinylpolypirrolidone, sodium starch glycolate, sodium croscarmellose, simethicone.

7. The water dispersible granulates according to anyone of claims from 1 to 6 wherein the percentage of Coenzyme Q₁₀, in its oxidized and/or reduced form, is comprised between 5 and 30% by weight.

8. The water dispersible granulates according to any one of claims from 1 to 7wherein the Coenzyme Q₁₀/surfactant(s) ratio is from 0,2 to 10.

9. The water dispersible granulates according to any one of claims from 1 to 8 wherein the Coenzyme Q₁₀/freely dissolving carrier(s) ratio is from 0,05 to 2.

10. A pharmaceutical or nutraceutical composition for oral administration containing a water dispersible powder granulate of claims 1-9 and a pharmaceutically or alimentary acceptable carrier.

11. A cosmetic product containing a water dispersible powder granulate according to anyone of claims 1-9 and a cosmetic acceptable carrier.

12. A process for the preparation of water dispersible solid granulates of claims 1-9, comprising the steps of:
a) adding a solution of Coenzyme Q₁₀ and of at least one surfactant in an organic solvent to a blended mixture of at least one freely soluble carrier, with the proviso that said surfactant is not a sucrose ester;
b) mixing and granulating;
c) evaporating the solvent and optionally calibrating and grinding to obtained the desired particle size.
